# EUROPEAN PATENT SPECIFICATION

(11) **EP 1 704 840 B1**
(45) Date of publication and mention of the grant of the patent: **16.01.2008**
(21) Application number: 06111283.5
(22) Date of filing: 16.03.2006
(51) Int. Cl.: A61F 9/007, A61M 5/32

(54) **Quick release tip assembly**
Spitzenanordnung mit Schnellverschluss
Arrangement d'une pointe de montage rapide

(30) Priority: 25.03.2005 US 89893
(43) Date of publication of application: 27.09.2006
(73) Proprietor: Alcon, Inc., 6331 Hünenberg (CH)
(72) Inventor: Ghannoum, Ziad R., Trabuco Canyon, CA 97679 (US)
(74) Representative: Hanna, Peter William Derek

(56) References cited:
- US-A- 4 169 984
- US-A- 4 442 836
- US-A- 4 911 161
- US-A- 5 002 537
- US-A- 5 222 937

## Description

### Background of the Invention

This invention relates generally to the field of cataract surgery and more particularly to a tip assembly for use on a liquefaction handpiece.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the less function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating curting tip liquifies or emulsifies the lens so that the lens may be aspirated out of the eye. The diseased lens, once removed, is replaced by an artificial lens.

Recently, a new cataract removal technique has been developed that involves the injection of hot (approximately 45°C to 105°C) water or saline to liquefy or gellate the hard lens nucleus, thereby making it possible to aspirate the liquefied lens from the eye. Aspiration is conducted with the injection of the heated solution and the introduction of a relatively cool irrigating solution, thereby quickly cooling and removing the heated solution. This technique is more fully described in U.S. Patent No. 5,616,120 (Andrew, et al.),

Handpiece and tips suitable for practicing this technique are described in U.S. Patent Nos. 5,989,212, 5,997,499, 6,080,128, 6,110,162, 6,179,805 and 6,206,848 (Sussman, et al.). These patents do not disclose any details of how to connect the tip to the handpiece, Current commercial embodiments of liquefaction handpieces use a threaded connection between the tip portion and the handpiece body.

Therefore, a need continues to exist for a quickly releasable assembly for connecting a tip to a handpiece.

### Brief Summary of the invention

The present invention provides a quick release tip assembly for a liquefaction handpiece, in accordance with claims which follow. The tip contains a lumen attached to a hub. The hub has a circumferential flange around its base. The handpiece contains a recess into which the hub fits. The bottom of the recess contains a plurality of movable snaps or catches that as sized to capture the flange on the tip hub. A slidable lever is connected to the snaps so that movement of the level causes the snaps to spread apart, thereby releasing the flange and allowing the tip to be easily removed from the handpiece.

Accordingly, one objective of the present invention is to pmvide a tip assembly for a liquefaction surgical handpiece.

Another objective of the present invention is to provide a quick release tip assembly for a liquefaction surgical handpiece.

These and other advantages and objectives of the present invention will become apparent from the detailed description and claims that follow.

### Brief Description of the Drawings

FIG. is an exploded partial cross-sectional view of the tip assembly of the present invention.

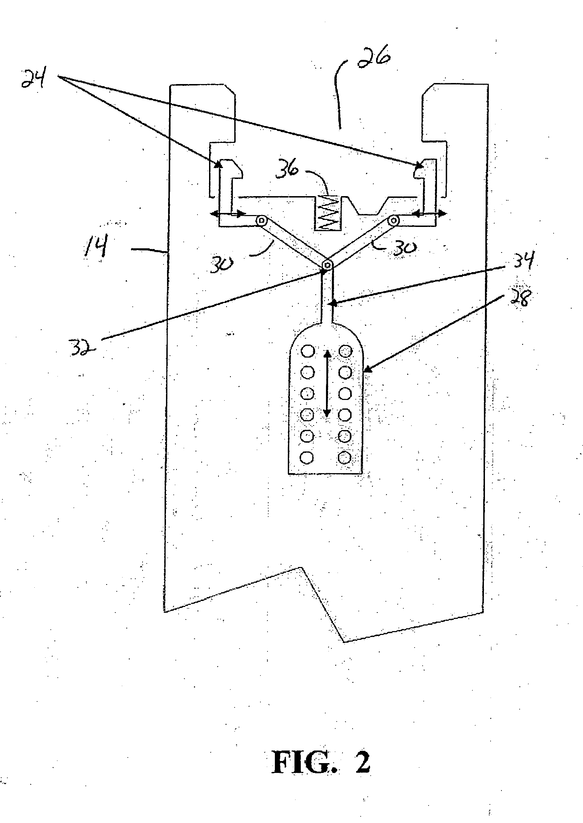

FIG. 2 is an exploded partial cross-sectional view of the tip assembly of the present invention highlighting the catch release mechanism.

### Detailed Description of the Invention

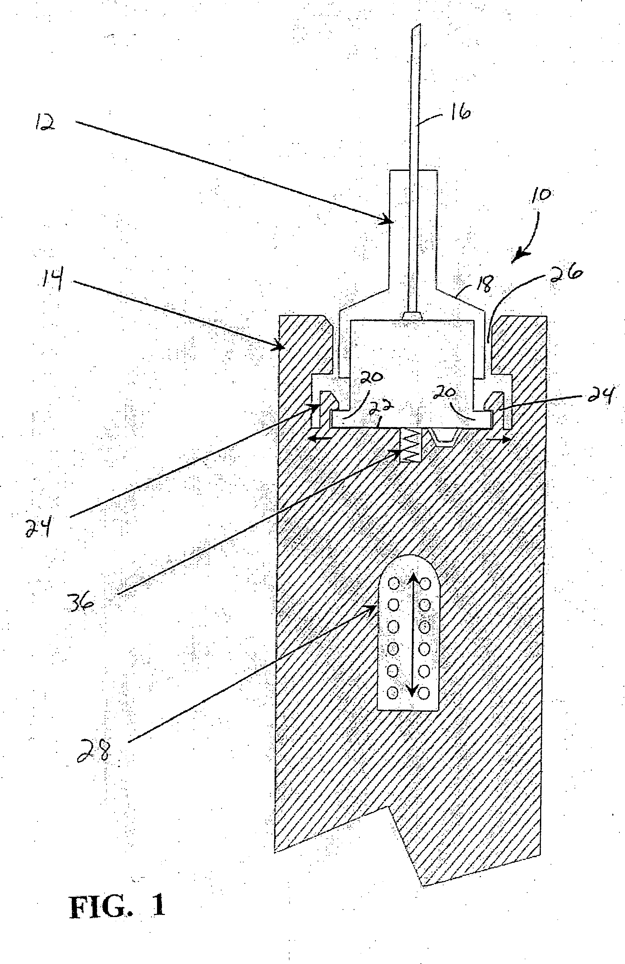

As best seen in FIG. 1, tip assembly 10 of the present invention generally includes tip 12 and handpiece 14. Tip 12 generally includes coaxial cannulae 16 that are connected to hub 1 S. Hub 18 includes a circumferential flange 20 about proximal base 22. Tip 12 may be made using conventional materials and techniques well-known in the art. Handpiece 14 is of conventional construction and contains recess 26, the bottom of which contains a plurality of movable snaps or catches 24. Recess 26 is sized and shaped to receive proximal base 22 of hub 18 so that calches 24 capture and hold flange 20 when hub 18 is inserted into recess 26.

As best seen in FIG. 2, catches 24 are connected internally within handpiece 14 to slidable lever 28 through rods 30, rotatable pin joints 32 and shaft 34, Shaft 34 and lever 28 may be made in a single piece, thereby constraining movement to oniy one direction. As shown by the arrows, vertical movement of lever 28 causes horizontal movement of catches 24. Spring 36 may also be located in recess 26 so that when hub 18 is nested within recess 26, spring 36 presses against base 22. Such biasing of spring 36 against base 22 assists in releasing hub 18 from catches 24 when catches 24 are pushed away from flange 20 by lever 28.

This description is given for purposes of illustration and explanation. It will be apparent to those skilled in the relevant art that changes and modifications may be made to the invention described above without departing from its scope as defined in the appended set of claims.

## Claims

1. A quick release tip assembly (10) for a liquefaction surgical handpiece (14), wherein the tip comprises a hub (18) with a flange (20) and **characterized in that** the handpiece comprises a recess (26) sized and shaped to receive the hub, and wherein a plurality of movable catches (24) are provided within the recess, the catches spaced to capture and hold the flange when the hub is inserted into the recess.

2. The tip assembly of claim 1, wherein the catches (24) are connected to a lever (28) so that movement of the lever causes movement of the catches (24).

3. The tip assembly of claim 2, wherein the lever is adapted to be slidable in a direction that causes the catches (24) to release the flange (20) when the hub is inserted into the recess.

4. The tip assembly of claim 1 or claim 2, further comprising a spring (36) located in the recess (26) pressing against the hub (18) when the hub is inserted into the recess.

## Patentansprüche

1. Spitzenaufbau (10) mit Schnellverschluß für ein chirurgisches Handstück (14) zur Verflüssigung, wobei die Spitze einen Hub (18) mit einem Flansch (20) aufweist und **dadurch gekennzeichnet ist, daß** das Handstück eine Vertiefung (26) aufweist, die eine Größe und Form besitzt, um den Hub aufzunehmen, und wobei mehrere bewegliche Haken (24) in der Vertiefung vorgesehen sind, wobei die Haken beabstandet sind, um den Flansch zu ergreifen und zu halten, wenn der Hub in die Vertiefung eingesetzt ist.

2. Spitzenanordnung nach Anspruch 1, wobei die Haken (24) mit einem Hebel (28) verbunden sind, so daß eine Bewegung des Hebels eine Bewegung der Haken (24) hervorruft.

3. Spitzenaufbau nach Anspruch 2, wobei der Hebel dafür eingerichtet ist, in einer Richtung gleitfähig zu sein, welche die Haken (24) dazu veranlaßt, den Flansch (20) zu lösen, wenn der Hub in die Vertiefung einsetzt ist.

4. Spitzenanordnung nach Anspruch 1 oder 2, die weiterhin eine Feder (36) aufweist, die sich in der Vertiefung (26) befindet und gegen den Hub (18) drückt, wenn der Hub in die Vertiefung eingesetzt ist.

## Revendications

1. Ensemble formant embout à libération rapide (10) pour une pièce à main chirurgicale de liquéfaction (14), dans lequel l'embout comporte un moyeu (18) muni d'une bride, et **caractérisé en ce que** la pièce à main comporte un évidement (26) dimensionné et mis en forme pour recevoir le moyeu, et dans lequel une pluralité de crochets de verrouillage mobiles (24) sont agencés dans l'évidement, les crochets de verrouillage étant espacés pour capturer et maintenir la bride lorsque le moyeu est inséré dans l'évidement.

2. Ensemble formant embout selon la revendication 1, dans lequel les crochets de verrouillage (24) sont reliés à un levier (28) de sorte qu'un déplacement du levier provoque un déplacement des crochets de verrouillage (24).

3. Ensemble formant embout selon la revendication 2, dans lequel le levier est adapté pour pouvoir être coulissé dans une direction qui amène les crochets de verrouillage (24) à libérer la bride (20) lorsque le moyeu est inséré dans l'évidement.

4. Ensemble formant embout selon la revendication 1 ou 2, comportant en outre un ressort (36) positionné dans l'évidement (26), appuyant contre le moyeu (18) lorsque le moyeu est inséré dans l'évidement.
